# EUROPÄISCHE PATENTSCHRIFT

(11) **EP 1 636 219 B1**
(45) Veröffentlichungstag und Bekanntmachung des Hinweises auf die Patenterteilung: **02.11.2006**
(21) Anmeldenummer: 04707508.0
(22) Anmeldetag: 03.02.2004
(51) Int. Cl.: C07D 417/06

(54) **NEUE THIAZOLYLMETHYL-PYRAZOLE, VERFAHREN ZU DEREN HERSTELLUNG SOWIE DEREN VERWENDUNG IN FÄRBEMITTELN FÜR KERATINFASERN**
NOVEL THIAZOLYLMETHYL PYRAZOLES, METHOD FOR THE PRODUCTION THEREOF, AND USE THEREOF IN DYES FOR KERATIN FIBERS
NOUVEAUX THIAZOLYLMETHYLE-PYRAZOLES, PROCEDE DE REALISATION ET UTILISATION DANS DES COLORANTS POUR FIBRES DE KERATINE

(30) Priorität: 27.05.2003 DE 10323970
(43) Veröffentlichungstag der Anmeldung: 22.03.2006
(73) Patentinhaber: Wella Aktiengesellschaft, 64274 Darmstadt (DE)
(72) Erfinder: GÖTTEL, Otto, CH-1723 Marly (CH); BRAUN, Hans-Jürgen, CH-3182 Ueberstorf (CH)
(86) Internationale Anmeldenummer: PCT/EP2004/000943
(87) Internationale Veröffentlichungsnummer: WO 2004/106332

(56) Entgegenhaltungen:
- EP-A- 0 891 971
- EP-A- 1 236 460
- WO-A-94/08969
- WO-A-94/08970

## Beschreibung

Gegenstand der vorliegenden Anmeldung sind neue 4,5-Diamino-pyrazole sowie deren physiologisch verträgliche Salze mit organischen oder anorganischen Säuren. Ein weiterer Gegenstand ist die Herstellung dieser Verbindungen sowie die Verwendung dieser Verbindungen als Farbstoff-Vorstufen in oxidativen Färbemitteln für Keratinfasern.

Auf dem Gebiet der traditionellen Haarfärbung haben Oxidationsfarbstoffe eine wesentliche kosmetische Bedeutung erlangt. Die Färbung entsteht hierbei durch Reaktion bestimmter Entwickler- und Kupplersubstanzen in Gegenwart eines Oxidationsmittels. Von besonderer Bedeutung sind nach wie vor Haarfärbemittel zur Färbung im Naturtonbereich. Daneben lassen sich durch Kombination geeigneter Oxidationsfarbstoff-Vorstufen auch zeitgemäß modische Farbnuancen erzeugen. Gegenwärtig im Modetrend liegen neben abgewandelten Naturtönen, wie beispielsweise Brauntönen mit ausgeprägten Aubergine- oder Kupfer-Nuancen, auch leuchtende Rottöne.

Neben der Erzeugung von Farbeffekten werden an Oxidationsfarbstoffe, die zur Behandlung menschlicher Haare vorgesehen sind, zunehmend höhere Anforderungen gestellt. Die Farbstoffe müssen einerseits in toxikologischer und dermatologischer Hinsicht unbedenklich sowie nicht sensibilisierend sein. Außerdem ist es erforderlich, dass durch Kombination geeigneter Entwickler- und Kupplerkomponenten eine breite Palette verschiedener Farbnuancen erzeugt werden kann.

Ferner wird für die erzielten Haarfärbungen eine gute Licht-, Schweiß-, Dauerwell-, Säure-, Basen- und Reibeechtheit gefordert. In jedem Fall müssen solche Haarfärbungen unter den heute üblichen Alltagsbedingungen mindestens vier bis sechs Wochen stabil bleiben.

Zur Abdeckung des zunehmend wichtigen Rotbereichs wurde bisher hauptsächlich 4-Aminophenol als Entwickler eingesetzt. Wegen Bedenken in Bezug auf die physiologische Verträglichkeit des 4-Aminophenols wurden als Alternative auch Pyrimidinderivate eingesetzt, die allerdings in färberischer Hinsicht nicht zufriedenstellen konnten. Schließlich wurden in der DE-A 42 34 885 und DE-A 42 34 887 Pyrazolderivate beschrieben, die intensive Rottöne lieferten.

Die vorgenannten Pyrazole ergeben überaus brillante und farbsatte Nuancen; infolge der hohen Deckkraft der Pyrazolfarbstoffe erhält man in der Regel jedoch sehr plakativ gefärbte Haare. Wenn allerdings nicht die satten Farben, sondern eher Farbreflexe des gleichen Farbtons gefragt sind, die sehr gut im Gegenlicht sichtbar werden -der Fachmann spricht hier von einer "transparenten Haarfarbe"- kann diesem Wunsch mit den bisher bekannten Pyrazolen kaum entsprochen werden. Der Grund hierfür liegt vor allem darin, dass Transparenz-Effekte nicht einfach dadurch erzielt werden können, dass man in den Farbmassen eine geringere Menge an Farbstoff-Vorstufen verwendet oder die Farbmassen stärker als üblich verdünnt. Mit dieser Maßnahme werden bei farbstarken Verbindungen meist sehr uneinheitliche und für den Kunden kaum zufriedenstellende Ergebnisse erzielt. In der Praxis zeigt sich dieses Problem im unterschiedlichen Aufziehverhalten von Farbstoffen, was beispielsweise durch die graduell unterschiedliche Haarbeschaffenheit zwischen ungeschädigtem Haaransatz und geschädigten Haarspitzen verursacht werden kann. Eine ungleichmäßige Anfärbung der Haare wird insbesondere dann beobachtet, wenn beim Waschen der Haare die Farbstoffe aus den stärker geschädigten Haarpartien dem Grad der Haarschädigung entsprechend stärker ausgewaschen werden. Zusammen mit dem in der Regel intakten Haaransatz wird im ungünstigen Fall ein unnatürliches, ungleichmäßiges und völlig unbefriedigendes Färbeergebnis erhalten.

Aufgabe der vorliegenden Erfindung ist es, die vorstehend beschriebene Problematik zu lösen und neue Farbstoffvorstufen für das oxidative Färbesystem zur Verfügung zu stellen, die neben einem guten Aufziehverhalten und einer guten Auswaschfestigkeit gleichzeitig "transparente Haarfärbungen" ermöglichen.

Es wurde nunmehr gefunden, dass bestimmte 4,5-Diaminopyrazole die vorstehende Aufgabe in hervorragender Weise erfüllen.

Gegenstand der Erfindung sind neue 4,5-Diaminopyrazole der allgemeinen Formel (I) 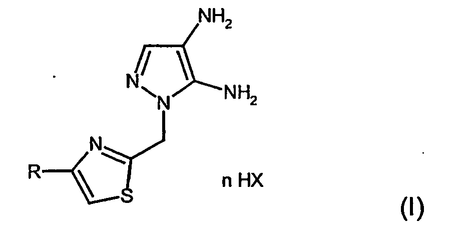 worin R eine geradkettige oder verzweigte eine C1-C6-Alkylgruppe, eine unsubstituierte Phenylgruppe oder eine einfach oder mehrfach substituierte Phenylgruppe darstellt, worin die Substituenten unabhängig voneinander ausgewählt werden können aus einem Halogenatom (F, Cl, Br, J), einer C1-C6-Carbonsäureestergruppe, einer geradkettigen oder verzweigten C1-C6-Alkoxygruppe (die gegebenenfalls durch ein oder zwei Hetero-atome unterbrochen sein kann), einer Hydroxyethoxygruppe, einer Dihydroxypropoxygruppe oder einer Nitrilgruppe.

Die erfindungsgemäßen Verbindungen lassen sich beispielsweise nach dem folgenden Schema 1 herstellen:

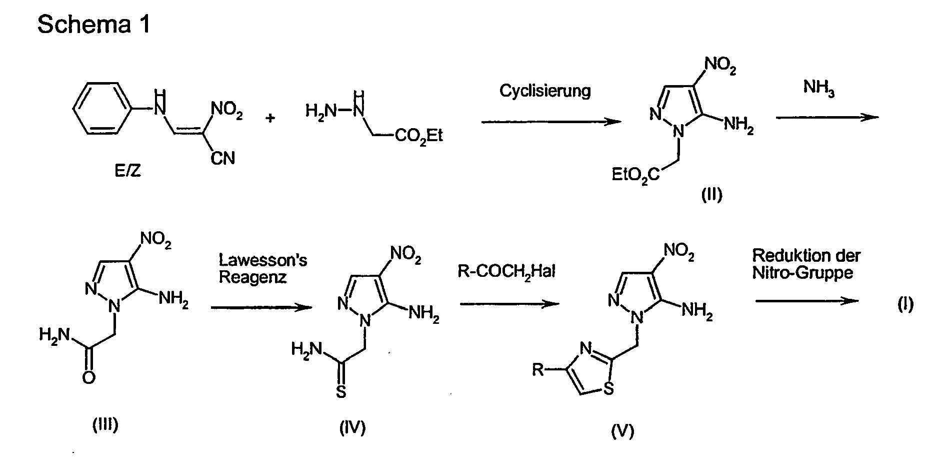

Dazu wird (2E/Z)-3-anilino-2-nitro-2-propennitril, hergestellt nach O. S. Wolfbeis, Chem. Ber. 114 (11), 3471 (1981), mit Hydrazino-essigsäure-ethylester cyclisiert und durch Aminolyse der Estergruppe die Carbonamidgruppe erzeugt. Anschließend erfolgt mittels Lawesson's Reagenz (= 2,4-Bis(4-methoxyphenyl)-1,3,2,4-dithiadiphosphetan-2,4-dithion) die Umwandlung zur Thioamid-Funktion, aus der sich die Seitenketten-Heterocyclen durch Umsetzung mit entsprechenden Halogenomethyl-arylketonen aufbauen lassen. Nach der Reduktion der Nitrogruppe am Pyrazolring können die erfindungsgemäßen Produkte isoliert werden.

Wegen der ausgeprägten Oxidationsempfindlichkeit der 4,5-Diaminopyrazole werden die Verbindungen in vorteilhafter Weise nicht als freie Basen, sondern als Säureaddukte isoliert. Die so erhaltenen Salze sind weitgehend oxidationsunempfindlich und ermöglichen dadurch eine einfachere Handhabung der Verbindungen.

Als Säuren können anorganische oder organische Säuren eingesetzt werden, wobei Salzsäure, Schwefelsäure, Phosphorsäure, Borsäure, Zitronensäure und Weinsäure bevorzugt sind. Besonders bevorzugt sind Salzsäure und Schwefelsäure.

Als Beispiele für geeignete Verbindungen der Formel (I) sind insbesondere zu nennen: 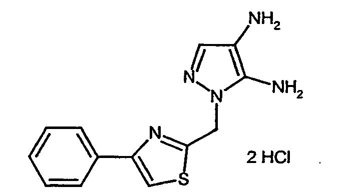 1-[(4-Phenyl-1,3-thiazol-2-yl)methyl]-4,5-diamino-1 H-pyrazol-Dihydrochlorid **(1a)** 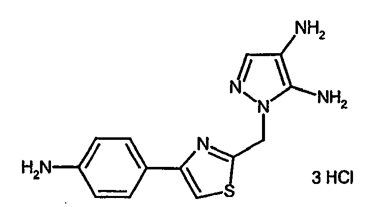 1-{[4-(4-Aminophenyl)-1,3-thiazol-2-yl]methyl}-4,5-diamino-1H-pyrazol-Trihydrochlorid **(1b)** 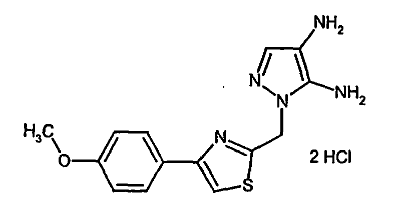 1-{[4-(4-Methoxyphenyl)-1,3-thiazol-2-yl]methyl}-4,5-diamino-1H-pyrazol-Dihydrochlorid **(1c)** 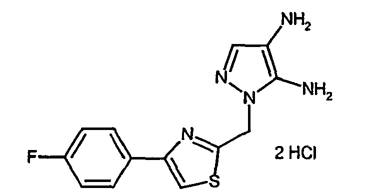 1-{[4-(4-Fluorophenyl)-1,3-thiazol-2-yl]methyl}-4,5-diamino-1H-pyrazol-Dihydrochlorid **(1d)** 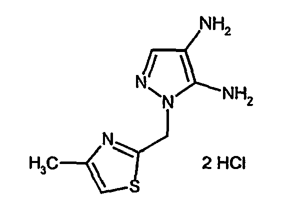 1-[(4-Methy)-1,3-thiazol-2-yl)methyl]-4,5-diamino-1H-pyrazol-Dihydrochlorid **(1e)** 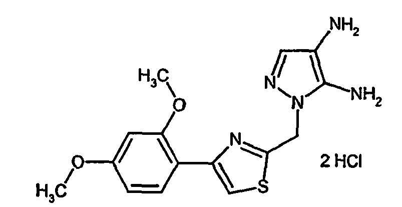 1-{[4-(2,4-Dimethoxyphenyl)-1,3-thiazol-2-yl]methyl}-4,5-diamino-1H-pyrazol-Dihydrochlorid **(1f)** 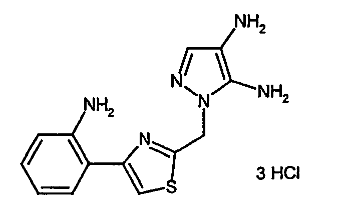 1-{[4-(2-Aminophenyl)-1,3-thiazol-2-yl]methyl}-4,5-diamino-1H-pyrazol-Trihydrochlorid **(1g)** 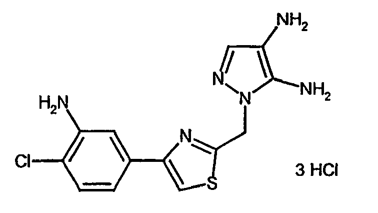 1-{[4-(3-Amino-4-chlorophenyl)-1,3-thiazol-2-yl]methyl}-4,5-diamino-1H-pyrazol-Trihydrochlorid **(1h)** 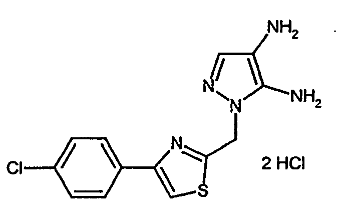 1-{[4-(4-Chlorophenyl)-1,3-thiazol-2-yl]methyl}-4,5-diamino-1H-pyrazol-Dihydrochlorid **(1i)** 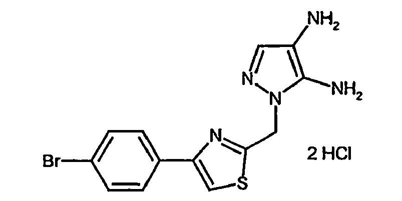 1-{[4-(4-Bromophenyl)-1,3-thiazol-2-yl]methyl}-4,5-diamino-1H-pyrazol-Dihydrochlorid **(1j)** 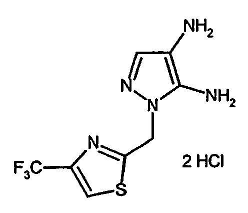 1-[(4-Trifluoromethyl-1,3-thiazol-2-yl)methyl]-4,5-diamino-1H-pyrazol-Dihydrochlorid **(1k)** 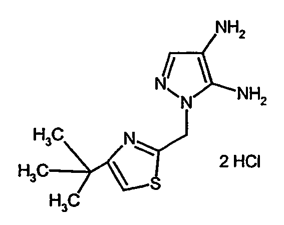 1-[(4-*t*-Butyl-1,3-thiazol-2-yl)methyl]-4,5-diamino-1H-pyrazol-Dihydrochlorid **(1l)**

Die Verbindungen der Formel (I) eignen sich hervorragend als Farbstoff-Vorstufen im oxidativen System zum Färben von Keratinfasern.

Ein weiterer Gegenstand der gegenwärtigen Erfindung ist daher die Verwendung der Verbindungen der Formel (I) in Färbemitteln für Keratinfasern, beispielsweise Wolle, Seide oder Haaren, insbesondere menschlichen Haaren.

Obwohl sich die Verbindungen der Formel (I) insbesondere für die Verwendung zur Färbung von Keratinfasern eignen, ist es prinzipiell auch möglich mit diesn Verbindungen andere natürliche oder synthetische Fasem, beispielsweise Baumwolle oder Nylon 66, zu färben.

Die Verbindung der Formel (I) kann sowohl alleine als auch in Kombination mit bestimmten bekannten Entwicklersubstanzen und/oder Kupplersubstanzen, die in oxidativen Färbesystemen zur Färbung von Fasermaterialien üblicherweise verwendet werden, eingesetzt werden.
Die Einsatzmenge der 4,5-Diaminopyrazole der Formel (I) beträgt in der Farbträgermasse etwa 0,01 bis 20 Gewichtsprozent, vorzugsweise etwa 0,1 bis 15 Gewichtsprozent.

Als geeignete Kupplersubstanzen können insbesondere genannt werden: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol, 7-Hydroxy-indol und 2,3-Indolindion, oder deren Salze.

Zur Herstellung von speziellen Nuancen oder dezenteren Tönen mit modischen Reflexen kann es besonders vorteilhaft sein, Verbindungen der Formel (I) in Kombination mit zusätzlichen Entwicklersubstanzen einzusetzen. Als Entwicklersubstanzen kommen Paraphenylendiamine, Paraaminophenole sowie weitere 4,5-Diamino-pyrazole oder deren Salze in Betracht. Insbesondere sind die folgenden Entwicklersubstanzen zu nennen:
1,4-Diamino-benzol (p-Phenylendiamin), 1,4-Diamino-2-methyl-benzol (p-Toluylen-diamin), 1,4-Diamino-2,6-dimethyl-benzol, 1,4-Diamino-2,5-dimethyl-benzol, 1,4-Diamino-2,3-dimethyl-benzol, 2-Chlor-1,4-diamino-benzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylaminophenol, 4-Amino-2-(aminomethyl)-phenol, 4-Amino-2-[(2-hydroxyethyl)-amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1 H)-pyrimidon, 4,5-Diamino-1-(2-hydroxyethyl)-1 H-pyrazol, 4,5-Diamino-1-(1-methylethyl)-1 H-pyrazol, 4,5-Diamino-1-[(4-methyl-phenyl)methyl]-1 H-pyrazol, 1-[(4-Chlorphenyl)methyl]-4,5-diamino-1H-pyrazol, 4,5-Diamino-1-methyl-1 H-pyrazol, 4,5-Diamino-3-methyl-1-phenyl-1 H-pyrazol, 4,5-Diamino-1-(2-hydroxyethyl)-3-methyl-1H-pyrazol, 1,2-Bis-(4,5-diamino-1H-pyrazol-1-yl)-ethan, 1,4-Bis-(4,5-diamino-pyrazol-1-yl-methyl)-benzol, 4,5-Diamino-1-(2-methylphenyl)-1 H-pyrazol, 4,5-Diamino-1-(3-methylphenyl)-1 H-pyrazol, 4,5-Diamino-1-(4-methylphenyl)-1 H-pyrazol, 4,5-Diamino-1-(2,4-dimethylphenyl)-1 H-pyrazol, 4,5-Diamino-1-(2,5-dimethylphenyl)-1 H-pyrazol, 4,5-Diamino-1-(2-ethylphenyl)-1H-pyrazol, 4,5-Diamino-1-(4-isopropylphenyl)-1 H-pyrazol, 4,5-Diamino-1-(4-methoxyphenyl)-1 H-pyrazol, 1-(4-Aminophenyl)-4,5-diamino-1 H-pyrazol, 1-(4-Chlorphenyl)-4,5-diamino-1 H-pyrazol, 4,5-Diamino-1-(2-pyridinyl)-1 H-pyrazol, 2-Amino-phenol, 2-Amino-6-methyl-phenol und 2-Amino-5-methyl-phenol, oder deren Salze.

Die Verbindungen der Formel (I) können selbstverständlich auch in Kombination mit üblichen direktziehenden anionischen, kationischen oder neutralen Farbstoffe verwendet werden. Zu den bevorzugten anionischen Farbstoffen zählen beispielsweise 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäuretrinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)amino]-2-phenylaminobenzolsulfonsäure-natriumsalz (Cl10385; Acid Orange No. 3), 4-[(2,4-Dihydroxyphenyl)azo]-benzolsulfonsäure-mononatriumsalz (Cl14270; Acid Orange No. 6), 4-[(2-Hydroxynaphth-1-yl)azo]-benzolsulfonsäurenatriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalinsulfonsäure-dinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (Cl45430;Acid Red No. 51), N-[6-(Diethylamino)-9-(2,4-disulfophenyl)-3H-xanthen-3-yliden]-N-ethylethanaminium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'-dihydroxyspiro[isobenzofuran-1 (3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (Cl45410; Acid Red No. 92), 3',6'-Dihydroxy-4',5'-diiodospiro-[isobenzofuran-1 (3H),9'(9H)-xanthen)-3-on-dinatriumsalz (Cl45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinondinatriumsalz (Cl 61570; Acid Green No. 25), Bis[4-(dimethylamino)-phenyl]-(3,7-disulfo-2-hydroxynaphth-1-yl)carbenium-inneres Salz, mononatriumsalz (Cl44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbeniuminneres salz, Calciumsalz (2:1) (CI42051; Acid Blue No. 3), 1-Amino-4-(cyclohexylamino)-9,10-anthrachinon-2-sulfonsäure-natriumsalz (C162045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäuredinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure natriumsalz Chrom-Komplex (Acid Red No. 195).

Zu den bevorzugten kationischen Farbstoffen zählen beispielsweise 9-(Dimethylamino)-benzo[a]phenoxazin-7-ium-chlorid (CI51175; Basic Blue No. 6), Di[4-(diethylamino)phenyl][4-(ethylamino)naphthyl]carbeniumchlorid (C142595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)amino]-1 (4H)-naphthalinon-chlorid (CI56059; Basic Blue No. 99), Bis[4-(dimethylamino)-phenyl][4-(methylamino)phenyl]carbenium-chlorid (CI42535; Basic Violet No. 1), Tris(4-amino-3-methylphenyl)-carbenium-chlorid (CI42520; Basic Violet No. 2), Tris[4-(dimethylamino)phenyl]carbenium-chlorid (C142555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphtholchlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)-azo]-pyrazol-5-on-chlorid (CI12719; Basic Yellow No. 57), Bis[4-(diethylamino)phenyl]phenylcarbenium-hydrogensulfat(1:1) (Cl42040; Basic Green No. 1).

Zum besseren Farbausgleich und zur Erzeugung von speziellen Nuancen besonders bewährt haben sich nichtionische Farbstoffe aus der Gruppe 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methyl-amino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)-azo)-4-methylphenol (CI11855; Disperse Yellow No. 3).

Aus der Gruppe der direktziehenden Farbstoffe besonders zu erwähnen sind 2-Amino-4,6-dinitrophenol, 2-Ethylamino-4,6-dinitrophenol, 2-[(2-Hydroxyethyl)amino]-4,6-dinitrophenol und Farbstoffe der allgemeinen Formel (II), 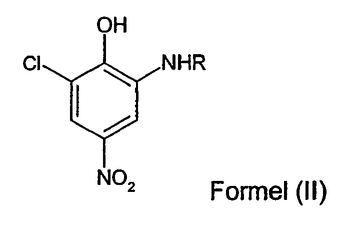 worin R Wasserstoff, Methyl, Ethyl oder Hydroxyethyl bedeutet.

Die vorstehend beschriebenen erfindungsgemäßen Kombinationen der Verbindungen der Formel (I) mit oxidativen Haarfarbvorstufen und/oder direktziehenden Farbstoffen werden zur Färbung in einer geeigneten Farbträgermasse appliziert.

Der Gegenstand der vorliegenden Erfindung betrifft weiterhin ein Mittel zur oxidativen Färbung von Haaren, das durch Vermischen einer Farbträgermasse mit einem Oxidationsmittel unmittelbar vor der Anwendung hergestellt wird und dadurch gekennzeichnet ist, dass es mindestens eine Verbindung der Formel (I) sowie gegebenenfalls weitere Farbstoffvorstufen und/oder direktziehende Farbstoffe enthält.

In diesem gebrauchsfertigen Färbemittel sind die Verbindungen der Formel (I) sowie die Farbvorstufen in einer Gesamtkonzentration von etwa 0,01 bis 10 Gewichtsprozent, vorzugsweise 0,2 und 6 Gewichtsprozent, enthalten. Die Gesamtkonzentration an direktziehenden Farbstoffen beträgt etwa 0,1 bis 10 Gewichtsprozent, vorzugsweise 0,1 bis 5 Gewichtsprozent.

Darüber hinaus können in der Farbträgermasse sowie dem Färbemittel noch Antioxidantien, Parfümöle, Komplexbildner, Netzmittel, Emulgatoren, Penetrationsmittel, Puffersysteme, Konservierungsstoffe, Verdicker, Pflegestoffe und andere kosmetische Zusätze vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze für Lösungen, Cremes, Emulsionen oder Gele sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionischen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet. Bezogen auf die Farbträgermasse sind dies zum Beispiel Netzmittel und Emulgatoren in Konzentrationen von etwa 0,5 bis 30 Gewichtsprozent, Verdicker in einer Menge von etwa 0,1 bis 25 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem flüssigen Oxidationsmittel unmittelbar vor der Anwendung hergestellt.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wässrigen Lösung in Betracht, wobei Wasserstoffperoxid besonders bevorzugt wird.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Der pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird. Der pH-Wert des gebrauchsfertigen Haarfärbemittels beträgt etwa 3 bis 11, vorzugsweise 6 bis 10,5.

Für die Einstellung des jeweiligen pH-Wertes der Farbträgermasse und des Oxidationsmittels können je nach dem gewünschten pH-Wert verdünnte organische oder anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge und Tris(hydroxymethyl)aminomethan, verwendet werden.

Nach der Vermischung der vorstehend beschriebenen Farbträgermasse mit dem Oxidationsmittel wird eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar aufgetragen.

Man läßt das erfindungsgemäße Haarfärbemittel etwa 10 bis 45 Minuten bei 15 bis 50 Grad Celsius, vorzugsweise 30 Minuten bei 40 Grad Celsius, auf das Haar einwirken und spült dann das Haar mit Wasser aus. Gegebenenfalls wird das Haar im Anschluß an diese Spülung mit einem Shampoo gewaschen und eventuell mit einer verdünnten schwachen organischen Säure, wie zum Beispiel Zitronensäure oder Weinsäure nachgespült. Abschließend wird das Haar getrocknet.

Die nachfolgenden Beispiele sollen den Gegenstand näher erläutern, ohne ihn jedoch auf diese Beispiele zu beschränken.

### Beispiele

### Beispiel 1: Herstellung der Thiazolyl-methyl-pyrazole der Formel (I)

### Stufe 1: (5 Amino-4-nitro-1H-pyrazol-1-yl)-essigsäureethylester (II)

Zu einer Suspension von 37,75 g (200 mmol) (2-E/Z)-3-anilino-2-nitro-2-propennitril und 46,36 g (300 mmol) Hydrazinoessigsäure-ethylesterhydrochlorid in 250 ml wasserfreiem Tetrahydrofuran gibt man bei 4°C unter Rühren 155 ml Diisopropylethylamin. Man rührt 1 Stunde lang bei dieser Temperatur weiter, gibt dann 130 ml Methanol zu und erhitzt anschließend für 18 Stunden unter Rückfluß. Nach dem Abkühlen werden ca. 200 ml abdestilliert, der Rückstand wird auf 500 ml gesättigte Natriumhydrogencarbonatlösung gegossen und das erhaltene Gemisch zweimal mit je 500 ml Essigester extrahiert. Nach dem Trocknen der vereinigten Essigesterphasen über wasserfreiem Magnesiumsulfat wird auf ca. 100 ml eingeengt und bis zur Kristallisation mit Hexan versetzt. Man läßt auskristallisieren, saugt ab, und trocknet im Vakuum bei 40 °C.
Ausbeute: 19,8 g (46%)
Mᵣ = 214,18 (C₇H₁₀N₄O₄)
MS (APCI⁺): 215 [M+H]⁺
¹H-NMR (DMSO-d₆) 7,93 (s, Pyrazol-H); 7,59 (s, NH₂); 4,89 (s, NCH₂); 4,16 (q, J = 7,1, OCH₂); 1,22 (*t*, J = 7,1, CH₃).

### Stufe 2: (5-Amino-4-nitro-1H-pyrazol-1-yl)-acetamid (III)

Zu einer Lösung von 17,3 g (80 mmol) des (5-Amino-4-nitro-1H-pyrazol-1-yl)-essigsäureethylesters aus Stufe 1 in 100 ml Methanol werden 100 ml 4N methanolische Ammoniaklösung zugegeben. Nach etwa 5 Minuten Rühren setzt Kristallisation ein. Man läßt über Nacht weiterrühren, saugt ab, wäscht mit wenig kaltem Methanol nach und trocknet im Vakuum bei 40 °C.
Ausbeute: 13,6 g (99%) farblose Kristalle
Mᵣ = 185,14 (C₅H₇N₅O₃)
MS (APCI⁺): 186 [M+H]⁺
¹H-NMR (DMSO-d₆): 7,88 (s, Pyrazol-H); 7,50, 7,47, 7,28 (3s, 2 NH₂); 4,60 (s, CH₂).

### Stufe 3: (5-Amino-4-nitro-1H-pyrazol-1-yl)-thioacetamid (IV)

Zu einer Lösung von 10 g (54 mmol) des (5-Amino-4-nitro-1H-pyrazol-1-yl)-acetamids aus Stufe 2 in 150 ml Tetrahydrofuran werden bei Raumtemperatur 26,2 g (65 mmol) Lawesson's Reagenz zugesetzt und die Reaktionsmischung unter Rückfluß erhitzt. Nach 1 Stunde wird die Reaktionsmischung abgekühlt, über Kieselgel filtriert und zur Trockne eingedampft. Der Rückstand wird in 100 ml Methylenchlorid aufgenommen und 30 Minuten lang bei Raumtemperatur gerührt, wobei das Produkt auskristallisiert. Absaugen des Rückstandes und Trocknen im Vakuum bei 40 °C ergeben 9,9 g eines blaßgelben Pulvers.
Mᵣ = 201,20 (C₅H₇N₅O₂S)
MS (APCl⁺): 202 [M+H]⁺
¹H-NMR (CD₃OD): 7,91 (s, Pyrazol-H); 4,97 (s, CH₂).

### Stufe 4: 4-Nitro-5-amino-1-thiazolyl-verbindungen der Formel (V)

Eine Lösung von 1 g (5 mmol) des (5-Amino-4-nitro-1H-pyrazol-1-yl)-thioacetamids aus Stufe 3 in 20 ml Tetrahydrofuran/ Isopropanol 1:1 wird mit 6 mmol Halogenomethyl-aryl/alkyl-keton versetzt und 5 Stunden lang unter Rückfluß erhitzt. Nach dem Abkühlen und Eindampfen der Reaktionsmischung wird im Zweiphasensystem, bestehend aus 20 ml gesättigter Natriumhydrogen-carbonat-Lösung und 20 ml Essigester, extrahiert. Die organische Phase wird abgetrennt und über wenig Magnesiumsulfat getrocknet. Es wird auf ca. 5 ml aufkonzentriert und durch Zugabe von Ether werden die jeweiligen Produkte auskristallisiert.

Die Verbindungen verhalten sich dünnschichtchromatographisch einheitlich. Nach dem Trocken im Vakuum bei 40°C erhält man die Nitroverbindungen der Formel (V) in reiner Form. Nachfolgend sind analytische Daten exemplarisch ausgewählter Nitroverbindungen der Formel (V) wiedergegeben:
**a)** R = Phenyl [C₁₃H₁₁N₅O₂S; Mᵣ=301,32]
   MS (APCl⁺): 302 [M+H]⁺
   ¹H-NMR (CDCl₃): 7,89 (s, 1 H); 7,86-7,82 (m, 2H); 7,53-7,39 (m, 4H); 6,76 (br. s, NH₂); 5,48 (s, CH₂)
**b)** R = 4-OMethyl-Phenyl [C₁₄H₁₃N₅O₃S; Mᵣ = 331,35]
   MS (ESI⁺): 332 [M+H]⁺
   ¹H-NMR (DMSO-d₆): 8,0 (s, 1 H); 7,89 (s, 1 H); 7,84, 6,98 (AB, J = 8,8, 4H); 7,71 (s, 2H); 5,61 (s, CH₂); 3,78 (s, OMe)
**c)** R = Methyl
   ¹H-NMR (DMSO-d₆): 7,98 (s, Pyrazol-H); 7,69 (s, NH₂); 7,24 (*q,* J = 1,0, Thiazol-H); 5,52 (s, CH₂); 2,34 (d, J = 1,0, Me)
**d)** R = 2-Nitro-phenyl [C₁₃H₁₀N₆O₄S; Mᵣ = 346,32]
   MS (ESI+): 347 [M+H]⁺
   ¹H-NMR (DMSO-d₆): 8,0, 7,98 (2s, 2H); 7,89-7,86, 7,79-7,56 (2m, 6H); 5,57 (s, CH₂).

### Stufe 5: Synthese der Thiazolyl-methyl-pyrazole der Formel (I)

Eine Lösung von 1 mmol der Nitroverbindungen der Formel (V) aus Stufe 4 wird in 5 ml einer 1:1-Mischung ausTetrahydrofuran/Isopropanol 6 Stunden lang an 50 mg 10% Pd/C bei 9 bar Wasserstoffdruck hydriert. Nach der Reduktion filtriert man über Kieselgur (Hyflo SuperCel) direkt in 2 ml 3,3 M ethanolische Salzsäure und läßt im Eisbad rühren. Die ausgefallenen Produkte sind farblos bis blaßrosa; man saugt ab und trocknet unter Sauerstoffausschluß im Vakuum. Die Ausbeuten liegen zwischen 60-85%.

Nachfolgend sind analytische Daten exemplarisch ausgewählter Thiazolyl-methyl-pyrazole der Formel (I) wiedergegeben:
**1a)** R= Phenyl [Hydrochlorid: C₁₃H₁₃N₅S•xHCl ; Mᵣ = 271,34 •xHCl]
   MS (APCI⁺): 272 [M+H]⁺
**1b)** R= 4- Aminophenyl [Hydrochlorid: C₁₃H₁₄N₆S •xHCl;
   Mᵣ = 286,35•xHCl]
   MS (APCI⁺): 287 [M+H]⁺, 205
   ¹H-NMR (D₂O): 7,93, 7,46 (AB, J = 8,7, 4H); 7,82 (s, 1H); 7,55 (s, 1 H); 5,56 ppm (s, CH₂)
**1c)** R= 4-Methoxyphenyl [Hydrochlorid: C₁₄H₁₅N₅OS•xHCl;
   Mᵣ = 301,37• xHCl]
   MS (ESI⁺): 302 [M+H]⁺
   ¹H-NMR (D₂O): 7,64, 6,95 (AB, J = 8,9, 4H); 7,55 (s, 1 H); 7,53 (s, 1 H); 5,52 (s, CH₂); 3,77 ppm (s, OMe)
**1d)** R= 4-Fluorophenyl [Hydrochlorid: C₁₃H₁₂FN₅S**·** xHCl;
   Mᵣ = 289,33• xHCI]
   MS (APCI⁺): 290 [M+H]⁺, 259
**1e)** R= Methyl [Hydrochlorid: C₈H₁₁N₅S• xHCl; Mᵣ = 209,27• xHCl]
   MS(ESI⁺): 210 [M+H]⁺
   ¹H-NMR (D₂O): 7,55 (s, 1 H); 7,37 (q, J = 1,0, 1 H); 5,62 (s, CH₂); 2,41 ppm (d, J = 1,0, Me)
**1f)** R= 2,4-Dimethoxyphenyl [Hydrochlorid: C₁₅H₁₇N₅O₂S• xHCl;
   Mᵣ = 331,39• xHCl]
   MS (APCI⁺): 332 [M+H]⁺, 301, 276, 250.
**1g)** R= 2-Aminophenyl [Hydrochlorid: C₁₃H₁₄N₆S • xHCl;
   Mᵣ = 286,35 • xHCl]
   MS (ESI⁺): 287 [M+H]⁺
   ¹H-NMR (D₂O): 7,95 (s, Pyrazol-H); 7,87-7,84 (m, Thiazol-H); 7,56-7,42 (m, 4H); 5,60 ppm (s, CH₂)
**1h)** R= 3-Amino-4-chlorophenyl [Hydrochlorid: C₁₃H₁₃CIN₆S• xHCl;
   Mᵣ = 323,82• xHCl
   MS (APCI+): 321, 323 [M+H]⁺

### Beispiel 2: Haarfärbemittel mit einem basischen pH-Wert

In 10 ml der nachfolgend angegebenen Basisrezeptur werden jeweils 0,25 mmol der in den nachfolgenden Tabellen 1-8 genannten Pyrazole der Formel (I) gelöst.

| | |
|---|---|
| 80,00 g | Ethanol |
| 100,00 g | Natriumlaurylethersulfat, 28%ige wässrige Lösung |
| 90,00 g | Ammoniak, 25%ige wässrige Lösung |
| 3,00 g | Ascorbinsäure |
| 4,00 g | Natriumsulfit |
| ad 1000,00 g | Wasser, entmineralisiert |

In gleicher Weise werden jeweils 0,25 mmol der in den nachfolgenden Tabellen 1-8 genannten Kupplersubstanzen in 10 ml der Basisrezeptur gelöst.

Unmittelbar vor der Anwendung werden jeweils 10 g Pyrazollösung mit 10 g Kupplerlösung miteinander vermischt. Zu dieser Mischung gibt man anschließend 20 g einer 6%igen Wasserstoffperoxid-Lösung und erhält nach dem Durchmischen die gebrauchsfertigen Färbelösungen.

Die so erhaltenen gebrauchsfertigen Oxidationshaarfärbemittel werden anschließend auf gebleichte Tierhaare aufgetragen. Nach einer Einwirkzeit von 30 Minuten bei 40°C werden die Haarsträhnen mit einem Farbpflegeshampoo gewaschen, mit Wasser gespült und getrocknet. Die erhaltenen Farbnuancen besitzen eine gut sichtbare Transparenz. Die entsprechenden L*a*b*-Werte sind in den folgenden Tabellen 1-8 zusammengefaßt.

**Tabelle 1: Entwickler = 1-[(4-Phenyl-1,3-thiazol-2-yl)methyl]-4,5-diamino-1 H-pyrazol-Dihydrochlorid (1a)**

| **Kuppler** | **L** | **a** | **b** | **Farbe** |
|---|---|---|---|---|
| 3-Aminophenol | 38,44 | 36,67 | 19,99 | rotviolett |
| 5-Amino-2-methyl-phenol | 50,30 | 39,89 | 42,33 | orange |
| 2-(2,4-Diaminophenoxy)-ethanol x 2HCl | 26,82 | 31,23 | 2,06 | violett |
| 3-Dimethylamino-phenyl-harnstoff | 32,56 | 20,94 | -25,81 | blau |

**Tabelle 2: Entwickler = 1-{[4-(4-Aminophenyl)-1,3-thiazol-2-yl]methyl}-4,5-diamino-1 H-pyrazol-Trihydrochlorid (1b)**

| **Kuppler** | **L** | **a** | **b** | **Farbe** |
|---|---|---|---|---|
| 3-Aminophenol | 33,96 | 35,54 | 17,61 | rotviolett |
| 5-Amino-2-methyl-phenol | 48,74 | 42,31 | 42,95 | orange |
| 2-(2,4-Diaminophenoxy)-ethanol x 2HCl | 26,41 | 30,54 | 1,54 | violett |
| 3-Dimethylamino-phenyl-harnstoff | 29,03 | 20,70 | -26,44 | blau |

**Tabelle 3: Entwickler = 1-([4-(4-Methoxyphenyl)-1,3-thiazol-2-yl]methyl}-4,5-diamino-1 H-pyrazol-Dihydrochlorid (1c)**

| **Kuppler** | **L** | **a** | **b** | **Farbe** |
|---|---|---|---|---|
| 3-Aminophenol | 44,45 | 33,90 | 18,43 | rotviolett |
| 5-Amino-2-methyl-phenol | 56,03 | 36,89 | 42,12 | orange |
| 2-(2,4-Diaminophenoxy)-ethanol x 2HCl | 32,51 | 30,50 | 2,53 | violett |
| 3-Dimethylamino-phenyl-harnstoff | 39,39 | 19,81 | -26,36 | blau |

**Tabelle 4: Entwickler = 1-{[4-(4-Fluorophenyl)-1,3-thiazol-2-yl]methyl)-4,5-diamino-1H-pyrazol-Dihydrochlorid (1d)**

| **Kuppler** | **L** | **a** | **b** | **Farbe** |
|---|---|---|---|---|
| 3-Aminophenol | 42,07 | 35,04 | 20,02 | rotviolett |
| 5-Amino-2-methyl-phenol | 55,24 | 40,35 | 45,24 | orange |
| 2-(2,4-Diaminophenoxy)-ethanol x 2HCl | 29,45 | 31,80 | 3,17 | violett |
| 3-Dimethylamino-phenyl-harnstoff | 35,89 | 21,56 | -27,26 | blau |

**Tabelle 5: Entwickler = 1-[(4-Methyl-1,3-thiazol-2-yl)methyl]-4,5-diamino-1H-pyrazol-Dihydrochlorid (1e)**

| **Kuppler** | **L** | **a** | **b** | **Farbe** |
|---|---|---|---|---|
| 3-Aminophenol | 34,13 | 40,31 | 20,95 | rotviolett |
| 5-Amino-2-methyl-phenol | 48,92 | 44,70 | 46,64 | orange |
| 2-(2,4-Diaminophenoxy)-ethanol x 2HCl | 25,19 | 34,96 | 4,32 | violett |
| 3-Dimethylamino-phenyl-harnstoff | 26,89 | 26,28 | -27,77 | blau |

**Tabelle 6: Entwickler = 1-{[4-(2,4-Dimethoxyphenyl)-1,3-thiazol-2-yl]methyl}-4,5-diamino-1H-pyrazol-Dihydrochlorid (1f)**

| **Kuppler** | **L** | **a** | **b** | **Farbe** |
|---|---|---|---|---|
| 3-Aminophenol | 50,44 | 36,12 | 18,51 | rotviolett |
| 5-Amino-2-methyl-phenol | 60,49 | 33,89 | 33,62 | orange |
| 2-(2,4-Diaminophenoxy)-ethanol x 2HCl | 38,18 | 29,19 | 7,31 | violett |
| 3-Dimethylamino-phenyl-harnstoff | 41,91 | 19,34 | -25,56 | blau |

**Tabelle 7: Entwickler = 1-{[4-(2-Aminophenyl)-1,3-thiazol-2-yl]methyl}-4,5-diamino-1H-pyrazol-Trihydrochlorid (1g)**

| **Kuppler** | **L** | **a** | **b** | **Farbe** |
|---|---|---|---|---|
| 3-Aminophenol | 32,44 | 36,75 | 18,81 | rotviolett |
| 5-Amino-2-methyl-phenol | 45,02 | 43,73 | 41,33 | orange |
| 2-(2,4-Diaminophenoxy)-ethanol x 2HCl | 23,50 | 30,27 | 2,67 | violett |
| 3-Dimethylamino-phenyl-harnstoff | 25,25 | 20,78 | -24,20 | blau |

**Tabelle 8: Entwickler = 1-{[4-(3-Amino-4-chlorophenyl)-1,3-thiazol-2-yl]methyl}-4,5-diamino-1H-pyrazol-Trihydrochlorid (1h)**

| **Kuppler** | **L** | **a** | **b** | **Farbe** |
|---|---|---|---|---|
| 3-Aminophenol | 48,93 | 36,51 | 20,57 | rotviolett |
| 5-Amino-2-methyl-phenol | 58,84 | 34,52 | 40,39 | orange |
| 2-(2,4-Diaminophenoxy)-ethanol x 2HCl | 38,25 | 26,95 | 3,27 | violett |
| 3-Dimethylamino-phenyl-harnstoff | 45,30 | 17,35 | -21,56 | blau |

Alle in der vorliegenden Anmeldung genannten Prozentangaben stellen - soweit nicht anders angegeben- Gewichtsprozente dar.

## Patentansprüche

1. 4,5-Diaminopyrazole der allgemeinen Formel (I) 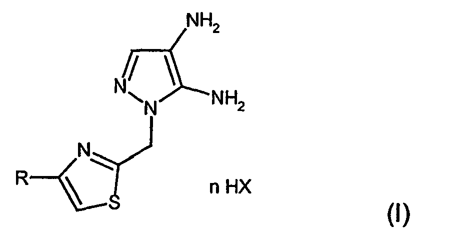 worin **R** eine geradkettige oder verzweigte eine C1-C6-Alkylgruppe, eine unsubstituierte Phenylgruppe oder eine einfach oder mehrfach substituierte Phenylgruppe darstellt, worin die Substituenten an der Phenylgruppe unabhängig voneinander ausgewählt werden können aus einem Halogenatom, einer C1-C6-Carbonsäureestergruppe, einer geradkettigen oder verzweigten C1-C6-Alkoxygruppe, einer durch ein oder zwei Heteroatome unterbrochenen geradkettigen oder verzweigten C1-C6-Alkoxygruppe, einer Hydroxyethoxygruppe, einer Dihydroxypropoxygruppe oder einer Nitrilgruppe.

2. 4,5-Diaminopyrazol gemäß Anspruch 1, **dadurch gekennzeichnet, dass** es ausgewählt ist aus 1-[(4-Phenyl-1,3-thiazol-2-yl)methyl]-4,5-diamino-1H-pyrazol-Dihydrochlorid, 1-{[4-(4-Aminophenyl)-1,3-thiazol-2-yl]methyl}-4,5-diamino-1H-pyrazol-Trihydrochlorid, 1-{[4-(4-Methoxyphenyl)-1,3-thiazol-2-yl]methyl}-4,5-diamino-1 H-pyrazol-Dihydrochlorid, 1-{[4-(4-Fluorophenyl)-1,3-thiazol-2-yl]methyl}-4,5-diamino-1H-pyrazol-Dihydrochlorid, 1-[(4-Methyl-1,3-thiazol-2-yl)methyl]-4,5-diamino -1 H-pyrazol-Dihydrochlorid, 1-{[4-(2,4-Dimethoxyphenyl)-1,3-thiazol-2-yl]methyl}-4,5-diamino-1H-pyrazol-Dihydrochlorid, 1-{[4-(2-Aminophenyl)-1,3-thiazol-2-yl]methyl}-4,5-diamino-1H-pyrazol-Trihydrochlorid, 1-{[4-(3-Amino-4-chlorophenyl)-1,3-thiazol-2-yl]methyl}-4,5-diamino-1H-pyrazol-Trihydrochlorid, 1-{[4-(4-Chlorophenyl)-1,3-thiazol-2-yl]methyl}-4,5-diamino -1 H-pyrazol-Dihydrochlorid, 1-{[4-(4-Bromophenyl)-1,3-thiazol-2-yl]methyl}-4,5-diamino-1H-pyrazol-Dihydrochlorid, 1-[(4-Trifluoromethyl-1,3-thiazol-2-yl)methyl]-4,5-diamino-1 H-pyrazol-Dihydrochlorid und 1-[(4-*t*-Butyl-1,3-thiazol-2-yl)methyl]-4,5-diamino-1 H-pyrazol-Dihydrochlorid.

3. Farbträgermasse auf der Basis von Oxidationsfarbstoffirorstufen, **dadurch gekennzeichnet dass** sie mindestens ein 4,5-Diamino-pyrazol der allgemeinen Formel (I) gemäß Anspruch 1 oder 2 enthält.

4. Farbträgermasse nach Anspruch 3, **dadurch gekennzeichnet, dass** sie das 4,5-Diamino-pyrazol der allgemeinen Formel (I) in einer Menge von 0,01 bis 20 Gewichtsprozent enthält.

5. Farbträgermasse nach Anspruch 3 oder 4, **dadurch gekennzeichnet, dass** sie zusätzlich mindestens eine Kupplersubstanz und/oder eine zusätzliche Entwicklersubstanz und/oder einen direktziehenden Farbstoff enthält.

6. Mittel zur Färbung von Keratinfasern, welches durch Vermischen einer Farbträgermasse mit einem Oxidationsmittel erhalten wird, **dadurch gekennzeichnet, dass** eine Farbträgermasse gemäß einem der Ansprüche 3 bis 5 verwendet wird.

7. Mittel nach Anspruch 6, **dadurch gekennzeichnet, dass** das Oxidationsmittel Wasserstoffperoxid ist.

8. Mittel nach Anspruch 6 oder 7, **dadurch gekennzeichnet, dass** die Farbträgermasse und das Oxidationsmittel in eine Gewichtsverhältnis von 5:1 bis 1:3 miteinander vermischt werden.

9. Mittel nach einem der Ansprüche 6 bis 8, **dadurch gekennzeichnet, dass** es ein Haarfärbemittel ist.

10. Verfahren zur Herstellung von 4,5-Diaminopyrazolen der Formel (I) gemäß Anspruch 1 oder 2, **dadurch gekennzeichnet, dass** (2E/Z)-3-anilino-2-nitro-2-propennitril mit Hydrazino-essigsäure-ethylester cyclisiert wird, sodann durch Aminolyse der Estergruppe die Carbonamidgruppe erzeugt wird, anschließend mittels Lawesson's Reagenz die Umwandlung zur Thioamid-Funktion erfolgt, aus der sich die Seitenketten-Heterocyclen durch Umsetzung mit entsprechenden Halogenomethyl-arylketonen aufbauen lassen und abschließend die Nitrogruppe am Pyrazolring reduziert wird.

## Claims

1. 4,5-Diaminopyrazoles of the general formula (I) 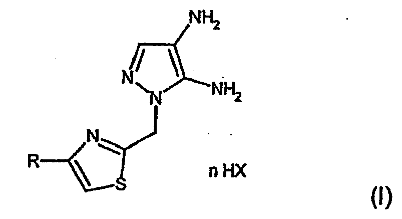 in which **R** is a straight-chain or branched C1-C6-alkyl group, an unsubstituted phenyl group or a mono- or polysubstituted phenyl group, in which the substituents on the phenyl group can be chosen, independently of one another, from a halogen atom, a C1-C6-carboxylic ester group, a straight-chain or branched C1-C6-alkoxy group, a straight-chain or branched C1-C6-alkoxy group interrupted by one or two heteroatoms, a hydroxyethoxy group, a dihydroxypropoxy group or a nitrile group.

2. 4,5-Diaminopyrazole according to Claim 1, **characterized in that** it is chosen from 1-[(4-phenyl-1,3-thiazol-2-yl)methyl]-4,5-diamino-1H-pyrazole dihydrochloride, 1-{[4-(4-aminophenyl)-1,3-thiazol-2-yl]methyl}-4,5-diamino-1H-pyrazole trihydrochloride, 1-{[4-(4-methoxyphenyl)-1,3-thiazol-2-yl]methyl}-4,5-diamino-1H-pyrazole dihydrochloride, 1-{[4-(4-fluorophenyl)-1,3-thiazol-2-yl]methyl}-4,5-diamino-1H-pyrazole dihydrochloride, 1-[(4-methyl-1,3-thiazol-2-yl)methyl]-4,5-diamino-1H-pyrazole dihydrochloride, 1-{[4-(2,4-dimethoxyphenyl)-1,3-thiazol-2-yl]methyl}-4,5-diamino-1H-pyrazole dihydrochloride, 1-{[4-(2-aminophenyl)-1,3-thiazol-2-yl]methyl}-4,5-diamino-1H-pyrazole trihydrochloride, 1-{(4-(3-amino-4-chlorophenyl)-1,3-thiazol-2-yl]methyl}-4,5-diamino-1H-pyrazole trihydrochloride, 1-{[4-(4-chlorophenyl)-1,3-thiazol-2-yl]methyl}-4,5-diamino-1H-pyrazole dihydrochloride, 1-{[4-(4-bromophenyl)-1,3-thiazol-2-yl]methyl}-4,5-diamino-1H-pyrazole dihydrochloride, 1-[(4-trifluoromethyl-1,3-thiazol-2-yl)methyl]-4,5-diamino-1H-pyrazole dihydrochloride and 1-[(4-t-butyl-1,3-thiazol-2-yl)methyl]-4,5-diamino-1H-pyrazole dihydrochloride.

3. Colour carrier mass based on oxidation dye precursors, **characterized in that** it comprises at least one 4,5-diaminopyrazole of the general formula (I) according to Claim 1 or 2.

4. Colour carrier mass according to Claim 3, **characterized in that** it comprises the 4,5-diaminopyrazole of the general formula (I) in an amount of from 0.01 to 20 per cent by weight.

5. Colour carrier mass according to Claim 3 or 4, **characterized in that** it additionally comprises at least one coupler substance and/or an additional developer substance and/or a direct dye.

6. Agent for dyeing keratin fibres which is obtained by mixing a colour carrier mass with an oxidizing agent, **characterized in that** a colour carrier mass according to one of Claims 3 to 5 is used.

7. Agent according to Claim 6, **characterized in that** the oxidizing agent is hydrogen peroxide.

8. Agent according to Claim 6 or 7, **characterized in that** the colour carrier mass and the oxidizing agent are mixed together in a weight ratio of from 5:1 to 1:3.

9. Agent according to one of Claims 6 to 8, **characterized in that** it is a hair dye.

10. Method for the production of 4,5-diaminopyrazoles of the formula (I) according to Claim 1 or 2, **characterized in that** (2E/Z)-3-anilino-2-nitro-2-propenenitrile is cyclized with ethyl hydrazino-acetate, then the carboxamide group is produced by aminolysis of the ester group, then by means of Lawesson's reagent, conversion to the thioamide function takes place from which the side chain heterocycles can be constructed through reaction with corresponding halomethyl aryl ketones and, finally, the nitro group on the pyrazole ring is reduced.

## Revendications

1. 4,5-diaminopyrazoles de formule générale (I) 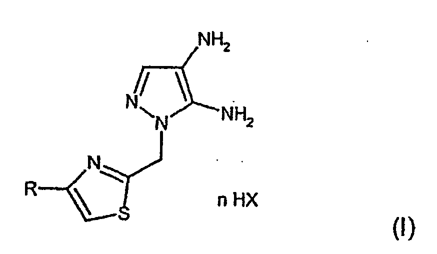 dans laquelle R représente un groupe alkyle en C₁-C₆ à chaîne droite ou ramifiée, un groupe phényle non substitué ou un groupe phényle une ou plusieurs fois substitué, les substituants sur le groupe phényle pouvant être choisis indépendamment les uns des autres parmi un atome d'halogène, un groupe ester d'acide carboxylique en C₁-C₆, un groupe alcoxy en C₁-C₆ à chaîne droite ou ramifiée, un groupe alcoxy en C₁-C₆ à chaîne droite ou ramifiée, interrompue par un ou deux hétéroatomes, un groupe hydroxyéthoxy, un groupe dihydroxypropoxy ou un groupe nitrilo.

2. 4,5-diaminopyrazole selon la revendication 1, **caractérisé en ce qu'**il est choisi parmi le dichlorhydrate de 1-[(4-phényl-1,3-thiazol-2-yl)-méthyl]-4,5-diamino-1H-pyrazole, le trichlorhydrate de 1-{[4-(4-aminophényl)-1,3-thiazol-2-yl]méthyl}-4,5-diamino-1H-pyrazole, le dichlorhydrate de 1-{[4-(4-méthoxyphényl)-1,3-thiazol-2-yl]méthyl}-4,5-diamino-1H-pyrazole, le dichlorhydrate de 1-{[4-(4-fluorophényl)-1,3-thiazol-2-yl]méthyl}-4,5-diamino-1H-pyrazole, le dichlorhydrate de 1-[(4-méthyl-1,3-thiazol-2-yl)-méthyl]-4,5-diamino-1H-pyrazole, le dichlorhydrate de 1-{[4-(2,4-diméthoxyphényl)-1,3-thiazol-2-yl]-méthyl}-4,5-diamino-1H-pyrazole, le trichlorhydrate de 1-{[4-(2-aminophényl)-1,3-thiazol-2-yl]méthyl}-4,5-diamino-1H-pyrazole, le trichlorhydrate de 1-{[4-(3-amino-4-chlorophényl)-1,3-thiazol-2-yl]méthyl}-4,5-diamino-1H-pyrazole, le dichlorhydrate de 1-{[4-(4-chlorophényl)-1,3-thiazol-2-yl]méthyl}-4,5-diamino-1H-pyrazole, le dichlorhydrate de 1-{[4-(4-bromophényl)-1,3-thiazol-2-yl]méthyl}-4,5-diamino-1H-pyrazole, le dichlorhydrate de 1-[(4-trifluorométhyl-1,3-thiazol-2-yl)méthyl]-4,5-diamino-1H-pyrazole et le dichlorhydrate de 1-[(4-tert-butyl-1,3-thiazol-2-yl)méthyl]-4,5-diamino-1H-pyrazole.

3. Matière colorante à base de précurseurs de colorants d'oxydation, **caractérisée en ce qu'**elle contient au moins un 4,5-diaminopyrazole de formule générale (I) selon la revendication 1 ou 2.

4. Matière colorante selon la revendication 3, **caractérisée en ce qu'**elle contient le 4,5-diaminopyrazole de formule générale (I) en une quantité de 0,01 à 20 % en poids.

5. Matière colorante selon la revendication 3 ou 4, **caractérisée en ce qu'**elle contient en outre au moins un coupleur et/ou un développeur supplémentaire et/ou un colorant direct.

6. Composition pour la teinture de fibres de kératine, qui est obtenue par mélange d'une matière colorante avec un oxydant, **caractérisée en ce qu'**on utilise une matière colorante selon l'une quelconque des revendications 3 à 5.

7. Composition selon la revendication 6, **caractérisée en ce que** l'oxydant est le peroxyde d'hydrogène.

8. Composition selon la revendication 6 ou 7, **caractérisée en ce que** la matière colorante et l'oxydant sont mélangés l'un avec l'autre en un rapport en poids de 5:1 à 1:3.

9. Composition selon l'une quelconque des revendications 6 à 8, **caractérisée en ce qu'**elle est une composition de teinture pour cheveux.

10. Procédé pour la préparation des 4,5-diaminopyrazoles de formule (I) selon la revendication 1 ou 2, **caractérisé en ce que** le (2E/Z)-3-anilino-2-nitro-2-propène-nitrile est cyclisé avec de l'hydrazino-acétate d'éthyle, puis le groupe carbamoyle est engendré par aminolyse du groupe ester, on effectue ensuite au moyen du réactif de Lawesson la conversion en la fonction thioamide, à partir de laquelle les hétérocycles des chaînes latérales peuvent se former par réaction avec des halogénométhyl-arylcétones correspondantes, et le groupe nitro sur le cycle pyrazole est finalement réduit.
